# EUROPÄISCHE PATENTSCHRIFT

(11) **EP 2 308 372 B1**
(45) Veröffentlichungstag und Bekanntmachung des Hinweises auf die Patenterteilung: **15.02.2017**
(21) Anmeldenummer: 09171978.1
(22) Anmeldetag: 01.10.2009
(51) Int. Cl.: A61B 5/053

(54) **Bioimpedanzmessvorrichtung**
Bioimpedance measuring device
Dispositif de mesure de la bio-impédance

(43) Veröffentlichungstag der Anmeldung: 13.04.2011
(73) Patentinhaber: seca ag, 4153 Reinach BL 1 (CH)
(72) Erfinder: Jensen, Björn, 22043, Hamburg (DE)
(74) Vertreter: Ahme, Johannes

(56) Entgegenhaltungen:
- EP-A2- 0 998 874
- JP-A- 2002 058 655
- US-A- 4 557 271
- US-A1- 2004 059 242
- US-B1- 6 280 396

## Beschreibung

Die vorliegende Erfindung betrifft eine Bioimpedanzmessvorrichtung zur Bestimmung von Zusammensetzungsdaten des menschlichen Körpers mit mehreren Elektroden und mit Messschaltungen einschließlich von Wechselstromquellen, Spannungsmessschaltungen und einer Steuer- und Auswerteeinheit, die dazu vorbereitet ist, nach Maßgabe von vorbestimmten Messprogrammen jeweils über zwei für das jeweilige Messprogramm spezifische Elektroden einen Wechselstrom aus den Wechselstromquellen in den Körper einzuprägen und mit zwei anderen Elektroden an verschiedenen Gliedmaßen mit den Spannungsmessschaltungen die resultierende Spannungen festzustellen und daraus die Impedanz von Körpersegmenten zu bestimmen, wobei zwei Handanlagekörper vorgesehen sind, die so geformt und mit jeweils zwei Elektroden versehen sind, dass ein Benutzer bei Anlegen seiner Hände mit jeder Hand in Kontakt mit zwei Elektroden an dem jeweiligen Handanlagekörper kommt, wobei jeder Handanlagekörper eine Handauflagefläche zur Auflage einer Handinnenfläche hat, wobei jede Handauflagefläche mit einem sich über einen Teil ihrer Länge erstreckenden, elektrisch isolierenden Trennsteg versehen ist, um bei auf der Handauflagefläche aufliegender Hand zwischen Mittel- und Ringfinger einzugreifen, und dass zu beiden Seiten des Trennstegs jeweils eine Elektrode mit einer Längserstreckung entlang der Handauflagefläche angeordnet ist.

Die Leitfähigkeit des menschlichen Körpers wird stark durch den Wasseranteil beeinflusst. Da die fettfreien Anteile des Körpers wie Muskeln und die Körperflüssigkeiten einen Großteil des körpereigenen Wassers beinhalten, während Fettgewebe einen nur relativ geringen Wasseranteil besitzt, kann durch die Bestimmung der Leitfähigkeit des Körpers oder eines Köpersegments (oder umgekehrt des Widerstands oder der Impedanz des Körpers oder des Körpersegments) ein Rückschluss auf den relativen Anteil von Fett gezogen werden, zumindest wenn weitere Daten wie die Größe und das Gewicht der Person berücksichtigt werden.

Eine Vorrichtung zur Bioimpedanzanalyse nach dem Oberbegriff von Anspruch 1 ist z.B. in WO 97/01303 beschrieben. Die beschriebene Vorrichtung weist acht Elektroden auf, nämlich vier Fußelektroden, jeweils zwei zum Kontaktieren eines Fußes, und vier Handelektroden, jeweils zwei zur Kontaktierung an einer Hand der Person. Dann wird ein Wechselstrom über jeweils zwei, an unterschiedlichen Gliedmaßen befindliche Elektroden eingeprägt und an zwei, ebenfalls an unterschiedlichen Gliedmaßen anliegenden Elektroden die Spannung gemessen. Durch Übergang zu anderen Paaren von stromeinprägenden Elektroden und spannungserfassenden Elektroden können sukzessive verschiedene Körpersegemente untersucht werden. Ferner kann bei Stromeinspeisung in eine Hand und einen Fuß und Spannungsmessung an derselben Hand und an demselben Fuß eine ganze Körperseite gemessen werden.

Aus DE 195 32 760 A1 ist eine Bioimpedanzmessvorrichtung bekannt, bei der jeweils zwei Klebeelektroden an Händen und Füßen anzubringen sind, wobei jeweils eine Elektrode etwas körperferner als die andere an dem jeweiligen der Gliedmaßen angebracht ist. Der Strom wird jeweils über die etwas weiter entfernt liegende Elektrode eingeprägt und die etwas näher am Rumpf liegende Elektrode die Spannung gemessen. Die Spannungsmessung findet daher in dem Strompfad statt, was zu Messfehlern führen kann, da die Ergebnisse von Variationen in der exakten Positionierung der Elektrode abhängen.

Bei der aus WO 97/01303 bekannten Bioimpedanzvorrichtung sind neben den Standflächen für die beiden Füße, an denen jeweils zwei Elektroden zur Kontaktierung der Fußsohlen vorgesehen sind, zwei Handgriffkörper jeweils zur Anlage einer Handinnenfläche des Benutzers vorgesehen. Jeder Handanlagekörper hat eine im Wesentlichen zylindrische Form, so dass er mit einer Hand umgreifbar ist, die so mit der Handinnenfläche und den Innenflächen der Finger in Kontakt mit der Oberfläche des Handanlagekörpers kommt. An dem Handanlagekörper sind jeweils zwei Elektroden so vorgesehen, nämlich eine im Bereich des Zylindermantels und eine an einer Stirnfläche des Zylinders, so dass bei Ergreifen des Handanlagekörpers durch den Benutzer die eine Elektrode in Kontakt mit der Handballenfläche oder der Fingerinnenfläche kommt und die andere Elektrode in Kontakt mit dem auf der Stirnfläche aufgelegten Daumen der Hand kommt. Dadurch wird zwar in gewissem Umfang durch Kontaktierung an Handfläche und Daumen eine Entkopplung von Strompfad und Spannungsmesspfad erreicht, die jedoch nicht zu einer sicher reproduzierbaren Messung der Impedanz führt. Tatsächlich ist der Daumen besonders beweglich, so dass die im Körper liegende Verbindungsstelle zwischen Strompfad und Spannungsmesspfad je nach Stellung des Daumens stark variiert. Daher sind erhebliche Abhängigkeiten der gemessenen Impedanzwerte von der Daumenstellung gegeben.

Aus JP 2002 058655 A ist eine Bioimpedanzmessvorrichtung mit den Merkmalen des Oberbegriffs von Anspruch 1 bekannt. Der im wesentlichen zylindrische Handauflagekörper ist mit zwei im wesentlichen um den gesamten Umfang verlaufenden Elektroden versehen. Zwischen den Elektroden liegt ein Trennsteg, der sich jedoch nur über einen kleinen Teil des Umfangs des Handauflagekörpers erstreckt, so dass über einen weiten Teil des Umfangs die beiden Elektroden ohne Trennsteg nebeneinanderliegen.

Es ist Aufgabe der vorliegenden Erfindung, eine Bioimpedanzmessvorrichtung so zu verbessern, dass sie bei einfacherer Handhabbarkeit durch den Benutzer besser reproduzierbare, präzisere Messwerte für die Impedanz liefern kann.

Zur Lösung dieser Aufgabe dienen die kennzeichnenden Merkmale des Patentanspruchs 1 in Verbindung mit dessen Oberbegriff. Vorteilhafte Ausführungsformen der Erfindung sind in den Unteransprüchen aufgeführt.

Erfindungsgemäß hat jeder Handanlagekörper eine Handauflagefläche zur Auflage einer Handinnenfläche. Jede Handauflagefläche ist mit einem sich über einen Teil ihrer Länge erstreckenden, elektrisch isolierenden Trennsteg versehen, um bei auf der Handauflagefläche aufliegender Hand zwischen Mittel- und Ringfinger einzugreifen. Auf beiden Seiten des Trennstegs und von diesem getrennt ist jeweils eine Elektrode mit einer Längserstreckung entlang der Handauflagefläche angeordnet, wobei der Trennsteg sich soweit über die Handauflagefläche erstreckt, dass er die Elektroden entlang deren gesamter Längserstreckung voneinander trennt, so dass bei auf der Handauflagefläche aufliegender Hand und in den Zwischenraum zwischen Mittel- und Ringfinger eingreifenden Trennsteg eine Elektrode nur in Kontakt mit kleinem Finger und/oder Ringfinger und die andere Elektrode nur in Kontakt mit Mittelfinger und/oder Zeigefinger kommen kann.

Auf diese Weise kommt es zu einer gut reproduzierbaren Positionierung der Handinnenfläche an dem Handanlagekörper, da der Trennsteg die Position der Hand daran festlegt. Ferner wird durch die elektrisch isolierende Eigenschaft des Trennstegs jegliche elektrische Wechselwirkung oder Kurzschlussmöglichkeit zwischen den beiden Elektroden vermieden.

Im Übrigen hat sich herausgestellt, dass durch die Kontaktierung der Elektroden an den Fingerinnenflächen in im Wesentlichen gleichem Abstand vom Rumpf des Benutzers die Stromeinspeisung durch die eine Elektrode und die Spannungsmessung durch die andere Elektrode an anatomisch vergleichbaren Körperpositionen erfolgt, so dass eine besser reproduzierbare und genauere Messung der Impedanz ohne gegenseitige Störung oder Beeinflussung von Strompfad und Spannungsmesspfad realisiert werden kann.

Grundsätzlich können auch mehrere Trennstege vorhanden sein, die dann zum Eingriff zwischen weiteren Fingerpaaren vorgesehen sind.

In einer bevorzugten Ausführungsform ist die Handauflagefläche im Wesentlichen als Ebene geformt, so dass die Hand flach aufgelegt werden kann, wobei über einen Teilbereich der Ebene der Trennsteg senkrecht vorsteht, um bei auf der Handauflagefläche aufgelegter Hand Mittel- und Ringfinger voneinander zu trennen, wobei zu beiden Seiten des Trennstegs jeweils eine Elektrode angeordnet ist.

In einer alternativen Ausführungsform ist die Handauflagefläche wenigstens teilweise gekrümmt geformt, so dass sie von einer Hand wie ein Handgriff umgriffen werden kann.

In einer bevorzugten Ausführungsform ist jede Elektrode mit einer sich im Wesentlichen parallel zu dem Trennsteg erstreckenden länglichen Erhebung versehen, die dazu gestaltet ist, um bei Auflage einer auf die Handauflagefläche aufliegenden Hand in Anlage an eine Fingerseitenfläche zu kommen. Dadurch wird bewirkt, dass elektrischer Kontakt auch zu einer Fingerseitenfläche hergestellt wird. Dadurch wird gewährleistet, dass die Elektrode auch mit der relativ dünnen Haut an einer Fingerseitenfläche in Kontakt kommt, was eine bessere reproduzierbare elektrische Kontaktierung sicherstellt als bei reinem Kontakt an der Handinnenfläche, die in der Regel dickere Hautbereiche und vielfach auch Hornhaut aufweist.

In einer besonders bevorzugten Ausführungsform ist jede Elektrode jeweils mit zwei im Wesentlichen parallel zu dem Trennsteg erstreckenden länglichen Fingermulden versehen, die dazu gestaltet sind, um in Kontakt mit den Fingerseitenflächen zweier aufgelegter Finger in den Fingermulden zu kommen. Dadurch wird eine besonders gute und reproduzierbare elektrische Kontaktierung an allen Fingerseitenflächen von kleinem Finger, Ringfinger, Mittelfinger und Zeigefinger gewährleistet.

Die Erfindung wird im Folgenden anhand von Ausführungsbeispielen in den Zeichnungen erläutert, in denen:
Fig. 1 eine perspektivische Ansicht eines Handanlagekörpers für eine erste Ausführungsform der Bioimpedanzmessvorrichtung zeigt,
Fig. 2 den Handanlagekörper aus Fig. 1 in perspektivischer Darstellung aus anderer Sicht zeigt,
Fig. 3 die Handhabung des Handanlagekörpers der Bioimpedanzmessvorrichtung aus Fig. 1 und 2 zeigt,
Fig. 4 einen Handanlagekörper einer alternativen Ausführungsform der Bioimpedanzmessvorrichtung zeigt, und
Fig. 5 eine Querschnittsansicht des Handanlagekörpers einer weiteren alternativen Ausführungsform der Bioimpedanzmessvorrichtung zeigt.

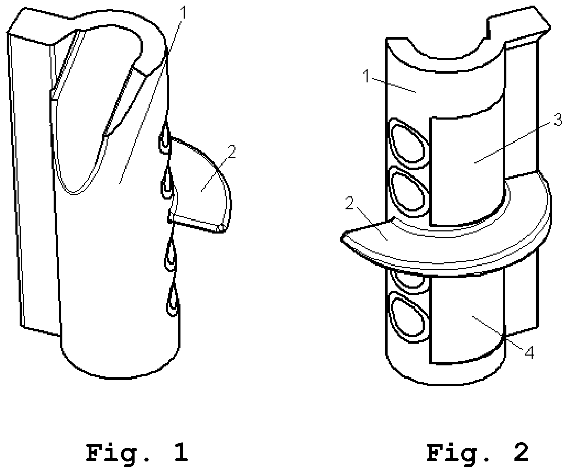

Fig. 1 bis 3 zeigen einen Handauflagekörper für eine erste Ausführungsform einer Bioimpedanzmessvorrichtung. Der Handanlagekörper hat eine teilweise gekrümmte, im Wesentlichen zylindrische Handauflagefläche, von der an einer Seite ein in Längsrichtung des Zylinders verlaufender Steg absteht. Ferner verläuft senkrecht zur Zylinderachse, etwa in der Mitte des zylindrischen Handanlagekörpers 1 ein sich etwa über die Hälfte des Zylindermantels erstreckender Trennsteg 2 aus elektrisch isolierendem Material.

Auf der Oberfläche des Handanlagekörpers 1 befindet sich in dem Bereich, der von dem Trennsteg 2 unterteilt wird, auf jeder Seite des Trennstegs 2 eine Elektrode 3 und 4. Die Elektroden 3 und 4 sind mit Wechselstromquellen, Spannungsmessschaltungen, Verstärkern (nicht gezeigt) verbunden, um nach einem vorgegebenen Messprogramm über bestimmte Elektroden einen Wechselstrom einzuprägen und über andere Elektroden die resultierende Spannung zu messen. Die Leitungen, die von dem Handanlagekörper 1 zu den weiteren Komponenten der Bioimpedanzmessvorrichtung (nicht gezeigt) führen sind ebenfalls nicht dargestellt.

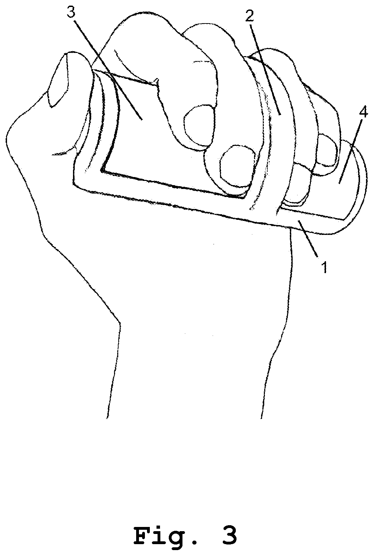

Fig. 3 zeigt die Handhabung des Handanlagekörpers 1. Dieser wird mit den Fingern so umgriffen, dass der in Längsrichtung des zylindrischen Körpers verlaufende Steg im Bereich der Handwurzel in Anlage kommt und der Handanlagekörper 1 dann von der Handinnenfläche und den Fingern umgriffen wird, so dass der Trennsteg 2 zwischen Mittel- und Ringfinger eingreift. Auf diese Weise wird jeder elektrische Kontakt zwischen den Elektroden 3 und 4, die in Anlage an den Innenflächen von Zeigefinger und Mittelfinger beziehungsweise Mittelfinger und kleinen Finger sind, vermieden. Gleichzeitig wird durch diese Anordnung von Elektroden 3, 4 und Trennsteg 2 an dem Handanlagekörper 1 eine weitgehend symmetrische Positionierung der beiden Elektroden 3 und 4 erreicht, so dass die Einprägung des Wechselstroms und die Messung von Spannungen an anatomisch äquivalenten Punkten des Körpers, nämlich auf gleicher Höhe an parallelen Fingerpaaren vorgenommen wird.

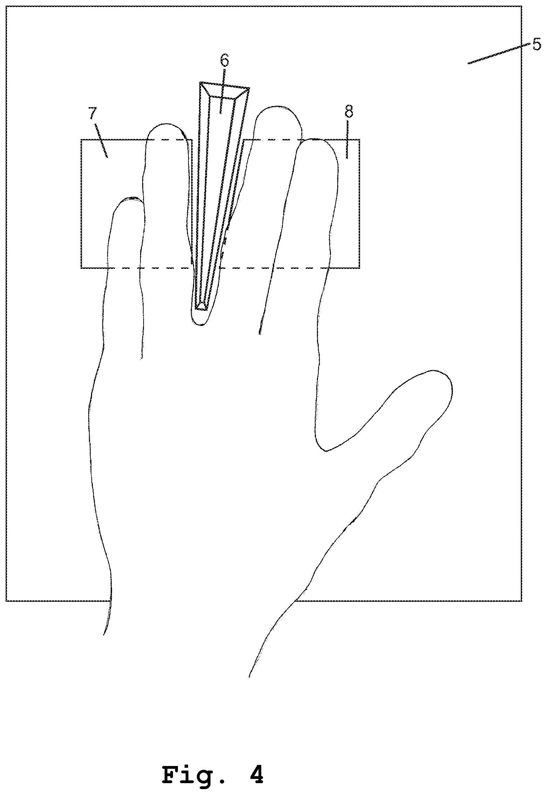

Fig. 4 zeigt einen Handanlagekörper 5 einer alternativen Bioimpedanzmessvorrichtung, der als ebener Handanlagekörper ausgebildet ist und zum Beispiel an einer Säule angebracht sein kann, die mit dem Fußteil der Messplattform mit der Standfläche für die Füße verbunden sein kann. Über einen Teil der Längsrichtung der Handauflagefläche des Handanlagekörpers 5 verläuft ein elektrisch isolierender Trennsteg 6. Zu beiden Seiten dessen und von dem Trennsteg 6 getrennt sind zwei Elektroden 7 und 8 auf der Handauflagefläche angeordnet. Auch bei dieser Gestaltung des Handanlagekörpers kommen jeweils zwei Finger elektrisch durch den Trennsteg 6 isoliert voneinander in Anlage an eine jeweilige der Elektroden 7 und 8, so dass auch hier eine anatomisch symmetrische oder gleichwertige Positionierung der Elektroden 7 und 8 realisiert wird.

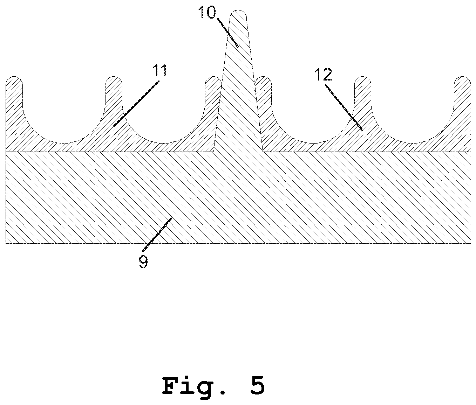

Fig. 5 zeigt einen Querschnitt in Richtung senkrecht zur Verlaufsrichtung des Trennstegs 10 durch den Handauflagekörper 9. Der Tennsteg 10 ist in diesem Fall einstückig mit dem Handanlagekörper 9 ausgebildet. Zu beiden Seiten des Trennstegs 10 sind jeweils eine Elektrode 11 und 12 angeordnet. Diese Elektroden sind mit im Wesentlichen parallel zur Verlaufsrichtung des Trennstegs 10 verlaufenden Erhebungen versehen, die dazu ausgestaltet sind, um in Kontakt mit einer Fingerseitenfläche zu kommen. In der dargestellten Ausführungsform sind jeweils drei Erhebungen an jeder Elektrode 11 und 12 vorgesehen, die jeweils Fingermulden für zwei Finger bilden. Bei dieser Ausführungsform kommt jeder Finger in einer der Mulden der Elektroden 11 und 12 in Anlage, so dass neben der Fingerinnenfläche auch die Fingerseitenflächen in elektrischen Kontakt mit der Elektrode kommen. Dadurch wird eine gut reproduzierbare elektrische Kontaktierung erreicht, da auch dünne und weiche Hautbereiche an den Fingerseiten in Kontakt kommen, während der Kontakt an den Fingerinnenseiten durch dickere Hautbereiche oder Hornhaut erschwert sein kann.

## Patentansprüche

1. Bioimpedanzmessvorrichtung zur Bestimmung von Zusammensetzungsdaten des menschlichen Körpers mit mehreren Elektroden und mit Messschaltungen einschließlich von Wechselstromquellen, Spannungsmessschaltungen und einer Steuer- und Auswerteeinheit, die dazu vorbereitet ist, nach Maßgabe von vorbestimmten Messprogrammen jeweils über zwei für das jeweilige Messprogramm spezifische Elektroden einen Wechselstrom aus den Wechselstromquellen in den Körper einzuprägen und mit zwei anderen Elektroden an verschiedenen Gliedmaßen mit den Spannungsmessschaltungen die resultierende Spannungen festzustellen und daraus die Impedanz von Körpersegmenten zu bestimmen, wobei zwei Handanlagekörper vorgesehen sind, die so geformt und mit jeweils zwei Elektroden (3, 4; 7, 8) versehen sind, dass ein Benutzer bei Anlegen seiner Hände mit jeder Hand in Kontakt mit zwei Elektroden an dem jeweiligen Handanlagekörper kommt, wobei jeder Handanlagekörper eine Handauflagefläche (1; 5) zur Auflage einer Handinnenfläche hat, wobei jede Handauflagefläche (1; 5) mit einem sich über einen Teil ihrer Länge erstreckenden, elektrisch isolierenden Trennsteg (2; 6) versehen ist, um bei auf der Handauflagefläche aufliegender Hand zwischen Mittel- und Ringfinger einzugreifen, und wobei zu beiden Seiten des Trennstegs jeweils eine Elektrode (3, 4; 7, 8) mit einer Längserstreckung entlang der Handauflagefläche angeordnet ist, **dadurch gekennzeichnet, dass** der Trennsteg (2; 6) sich soweit über die Handauflagefläche erstreckt, dass er die zwei Elektroden (3, 4; 7, 8) eines Handanlagekörpers entlang deren gesamter Längserstreckung voneinander trennt, so dass bei auf der Handauflagefläche aufliegender Hand und in den Zwischenraum zwischen Mittel- und Ringfinger eingreifendem Trennsteg (2; 6) eine der zwei Elektroden nur in Kontakt mit kleinem Finger und/oder Ringfinger der Hand und die andere der zwei Elektroden nur in Kontakt mit Mittelfinger und/oder Zeigefinger derselben Hand kommen kann.

2. Bioimpedanzmessvorrichtung nach Anspruch 1, wobei die Handauflagefläche (5) im Wesentlichen als Ebene geformt ist.

3. Bioimpedanzmessvorrichtung nach Anspruch 1, wobei die Handauflagefläche (1) wenigstens teilweise gekrümmt geformt ist, so dass sie von einer Hand umgreifbar ist.

4. Bioimpedanzmessvorrichtung nach einem der vorhergehenden Ansprüche, wobei jede Elektrode mit einer sich im Wesentlichen parallel zu dem Trennsteg (10) erstreckenden länglichen Erhebung (11, 12) versehen ist die dazu gestaltet ist, um bei Auflage einer auf der Handauflagefläche aufliegenden Hand in Anlage an eine Fingerseitenfläche zu kommen.

5. Bioimpedanzmessvorrichtung nach Anspruch 4, wobei jede Elektrode jeweils mit zwei sich im Wesentlichen parallel zu dem Trennsteg (10) erstreckenden länglichen Fingermulden versehen ist, die dazu gestaltet sind, um in Kontakt mit den Fingerseitenflächen zwei aufgelegten Finger zu kommen.

## Claims

1. Bioelectrical impedance measuring apparatus for determining composition data of a human body having a plurality of electrodes and measuring circuitry, including alternating current sources, voltage measuring circuits and a control and analysis unit, which control and analysis unit is arranged to inject an alternating current from the alternating current source, based on predetermined measuring programs, through two electrodes into the body, which electrodes are specific for the specific measuring program, and to determine with two other electrodes on different limbs the resulting voltages using the voltage measuring circuits, and to determine therefrom the impedance of body segments, wherein two hand contact bodies are provided, each of which is shaped in such a manner and provided with two electrodes (3, 4; 7, 8) in such a manner that a user when contacting the hand contact bodies with his hands comes into contact with two electrodes for each hand at the respective hand contact body, wherein each hand contact body has a hand seating surface (1; 5) for placement of a hand inner surface thereon, wherein each hand seating surface (1; 5) is provided with an electrically insulating separating wall (2; 6) extending over a part of the length of the hand seating surface, the separating wall being adapted to project into the space between middle and ring finger when a hand is placed on the hand seating surface, and wherein on both sides of the separating wall an electrode (3, 4; 7, 8) having a longitudinal extension along the hand seating surface is provided, **characterized in that** the separating wall (2; 6) extends so far along the hand seating surface that it separates the two electrodes (3, 4; 7, 8) of a hand contact body along their entire longitudinal extension such that, when a hand is placed on the hand seating surface and the separating wall (2; 6) is projecting into the space between middle and ring finger, one of the two electrodes can come into contact with the small finger and/or ring finger only and the other electrode can come into contact with middle finger and/or index finger only.

2. Bioelectrical impedance measuring apparatus according to claim 1, **characterized in that** the hand seating surface (5) is essentially formed as a plane.

3. Bioelectrical impedance measuring apparatus according to claim 1, **characterized in that** the hand seating surface (1) is at least partially curved such that it may be grasped by a hand.

4. Bioelectrical impedance measuring apparatus according to claim 1, **characterized in that** each electrode is provided with a projection which extends essentially parallel to the separating wall (10) and which is adapted in order to, when a hand is placed on the hand seating surface, abut against a side surface of a finger.

5. Bioelectrical impedance measuring apparatus according to claim 4, **characterized in that** each electrode is provided with two elongated finger depressions or troughs extending essentially parallel to the separating wall (10), said finger depressions or troughs being adapted to establish contact to finger side surfaces of two fingers placed therein.

## Revendications

1. Dispositif de mesure de bioimpédance pour la détermination de données de composition du corps humain avec plusieurs électrodes et avec des circuits de mesure, y compris des sources de courant alternatif, des circuits de mesure de tension et une unité de commande et d'évaluation, qui est préparée afin d'appliquer conformément à des programmes de mesure prédéterminés respectivement sur deux électrodes spécifiques pour le programme de mesure respectif un courant alternatif provenant des sources de courant alternatif dans le corps et de constater les tensions résultantes avec deux autres électrodes sur différents membres avec les circuits de mesure de tension et à partir de là déterminer l'impédance de segments de corps, dans lequel deux corps d'installation de main sont prévus, lesquels sont formés et sont pourvus de respectivement deux électrodes (3, 4 ; 7, 8) de sorte qu'un utilisateur vienne en contact lors de la pose de ses mains avec chaque main avec deux électrodes sur le corps d'installation de main respectif, dans lequel chaque corps d'appui de main a une surface d'appui de main (1 ; 5) pour l'appui d'une surface intérieure de main, dans lequel chaque surface d'appui de main (1 ; 5) est pourvue d'une nervure de séparation (2 ; 6) électroisolante, s'étendant sur une partie de sa longueur, afin d'intervenir en cas de main posée sur la surface d'appui de main entre le majeur et l'annulaire, et dans lequel des deux côtés de la nervure de séparation, respectivement une électrode (3, 4 ; 7, 8) est agencée avec une étendue longitudinale le long de la surface d'appui de main, **caractérisé en ce que** la nervure de séparation (2 ; 6) s'étend sur la surface d'appui de main tant qu'elle sépare l'une de l'autre les deux électrodes (3, 4 ; 7, 8) d'un corps d'installation de main le long de son étendue longitudinale entière de sorte qu'en cas de main posée sur la surface d'appui de main et de nervure de séparation (2 ; 6) s'engageant dans l'espace intermédiaire entre le majeur et l'annulaire, une des deux électrodes puisse venir seulement en contact avec le petit doigt et/ou l'annulaire de la main et l'autre des deux électrodes puisse venir seulement en contact avec un majeur et/ou index de la même main.

2. Dispositif de mesure de bioimpédance selon la revendication 1, dans lequel la surface d'appui de main (5) est formée sensiblement comme un plan.

3. Dispositif de mesure de bioimpédance selon la revendication 1, dans lequel la surface d'appui de main (1) est formée au moins en partie de manière courbée de sorte qu'elle puisse être entourée par une main.

4. Dispositif de mesure de bioimpédance selon l'une quelconque des revendications précédentes, dans lequel chaque électrode est pourvue d'une élévation (11, 12) oblongue s'étendant sensiblement parallèlement à la nervure de séparation (10), laquelle élévation est conçue afin de venir en appui contre une surface latérale de doigt en cas de pose d'une main posée sur la surface d'appui de main.

5. Dispositif de mesure de bioimpédance selon la revendication 4, dans lequel chaque électrode est pourvue respectivement de deux creux de doigt oblongs s'étendant sensiblement parallèlement à la nervure de séparation (10), qui sont conçus afin de venir en contact avec les surfaces latérales de doigt de deux doigts posés.
